(19) Europäisches Patentamt
European Patent Office
Office européen des brevets

(11) Veröffentlichungsnummer: **0 185 872**
**B1**

# (12) EUROPÄISCHE PATENTSCHRIFT

(45) Veröffentlichungstag der Patentschrift: **07.02.90**

(21) Anmeldenummer: **85113226.6**

(22) Anmeldetag: **18.10.85**

(51) Int. Cl. [5]: **C 07 C 33/20**, C 07 C 33/30, C 07 C 69/157, A 61 K 7/46

## (54) 1-Phenyl-2-methyl-3-hydroxy-(3-acyloxy)-alkylverbindungen, und deren Verwendung als Riechstoffe.

(30) Priorität: **26.10.84 DE 3439203**

(43) Veröffentlichungstag der Anmeldung: **02.07.86 Patentblatt 86/27**

(45) Bekanntmachung des Hinweises auf die Patenterteilung: **07.02.90 Patentblatt 90/06**

(84) Bennante Vertragsstaaten: **BE CH DE FR GB LI NL**

(56) Entgegenhaltungen:
**EP-A-0 032 576**
**CH-A-628 315**
**DE-A-2 439 294**
**DE-A-3 103 268**
**GB-A-1 250 291**
**US-A-4 524 021**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft**
**Carl-Bosch-Strasse 38**
**D-6700 Ludwigshafen (DE)**

(72) Erfinder: **Gramlich, Walter, Dr.**
**Auf der Hoehe 11**
**D-6803 Edingen-Neckarhausen (DE)**
Erfinder: **Himmele, Walter, Dr.**
**Eichenweg 14**
**D-6909 Walldorf (DE)**
Erfinder: **Hoffmann, Werner, Dr.**
**Fritzsche Dodge & Olcott Inc. 76 Ninth Avenue**
**New York, N.Y. 10113-0009 (US)**

EP 0 185 872 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBERGRAF, STOCKHOLM 1990

**Beschreibung**

Die Erfindung betrifft 1-Phenyl-2-methyl-3-hydroxy-alkylverbindungen der allgemeinen Formel I

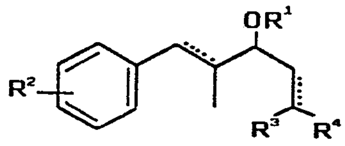

(I),

in der $R^1$ für Wasserstoff oder eine Acylgruppe mit 1 bis 5 C-Atomen, vorzugsweise eine Acetylgruppe steht, $R^2$, $R^3$ und $R^4$ gleich oder verschieden sind und für Wasserstoff, eine $C_1$-$C_5$-Alkyl- oder -Alkoxygruppe stehen, wobei die Reste $R^1$, $R^2$, $R^3$ und $R^4$ zusammen maximal 8 C-Atome enthalten sollen, und eine oder beide gestrichelten Linien für eine weitere Bindung stehen können sowie diese Verbindungen enthaltende Riechstoff-Kompositionen und Verwendung dieser Verbindungen als Riechstoffe bzw. als Bestandteile von Riechstoff-Kompositionen.

Bei der Herstellung von Verbindungen der Formel I als Zwischenprodukte wurde überraschend festgestellt, daß diese Verbindungen einen wunderbaren blumigen Duft aufweisen, der sich zudem noch durch große Haftfestigkeit auszeichnet. Da darüberhinaus der Duft auch in Anwesenheit von Luft und Licht über Monate angenehm und frisch bleibt und die Verbindungen auch auf relativ einfache Weise hergestellt werden können, sind sie hervorragend als Riechstoffe geeignet. Sie können für sich allein als Riechstoffe oder aber als Bestandteil von Riechstoff-Kompositionen verwendet werden.

Als als Riechstoffe bekannte aromatische Alkohole bzw. Acetate seien beispielsweise genannt: 2-Phenylethanol mit seinem Rosengeruch, 1-Phenyl-ethylacetat mit einem herbgrünen Blütengeruch, 3-(4-tert.-Butyl-phenyl)-2-methyl-propanal sowie Cyclamenaldehyd, welche beide Blütendüfte vom Typ Maiglöckchen bzw. Lilien aufweisen. Im Vergleich zu den genannten bekannten Duftstoffen verfügen die erfindungsgemäßen Verbindungen über einen länger anhaftenden und länger frischen Blütenduft.

Eine Zusammenfassung über Riechstoffe mit fruchtigen, blumigen und grünen Duftnoten findet sich beispielsweise in der Chemiker-Zeitung 107, Seiten 317 - 25.

Die Herstellung der neuen Verbindungen erfolgt in an sich bekannter Weise, so daß sich nähere Einzelheiten erübrigen und wir uns auf prinzipielle Ausführungen beschränken können.

So erhält man beispielsweise Verbindungen vom Typ Ia

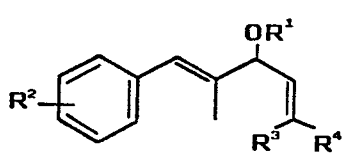

(Ia),

indem man einen Aldehyd der Formel IIa in einer Grignard-Reaktion mit einem Vinylmagnesiumhalogenid III umsetzt,

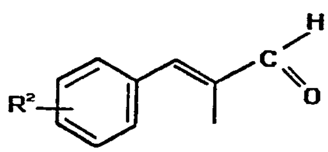

(IIa),

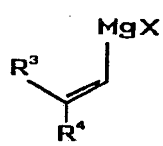

(III),

anschließend hydrolysiert und den erhaltenen Alkohol gegebenenfalls mit einem Carbonsäureanhydrid oder Carbonsäurechlorid verestert.

Anstelle der Grignard-Vinylierung kann man auch den Aldehyd IIa äthinylieren und anschließend mit einem Lindlar-Katalysator partiell zu Ia hydrieren.

Verwendet man anstelle eines Lindlar-Katalysators einen aktiveren Palladium-Katalysator, so erhält man die entsprechenden gesättigten Verbindungen der Formel Ib

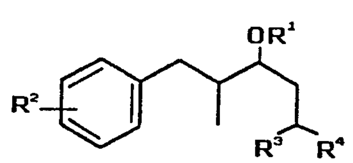

(Ib),

Bei der entsprechenden Grignard-Reaktion eines Aldehyds der Formel IIa mit einem gesättigten Alkylmagnesiumhalogenid der Formel IV

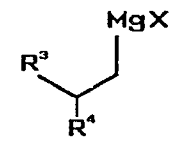

(IV),

erhält man Alkohole der Formel Ic; bei Grignard-Reaktion eines Aldehyds der Formel IIb

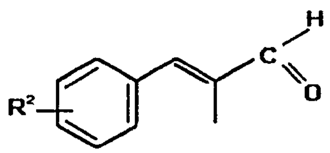

(IIb)

mit einem Vinylmagnesiumhalogenid der Formel III Alkohole der Formel Id und bei Grignard-Umsetzung eines Aldehyds der Formel IIb mit einem Alkylmagnesiumhalogenid IV wiederum Alkohole der Formel Ib.

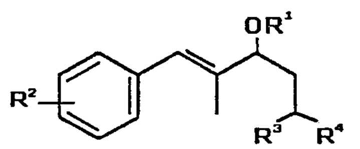

(Ic)

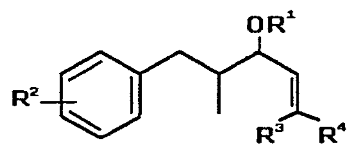

(Id)

Die Alkohole können dann anschließend mit entsprechenden Carbonsäurehalogeniden oder -anhydriden zu den entsprechenden Acylverbindungen Ia bis Id umgesetzt werden. Die gesättigten Acyloxyverbindungen Ib können aber auch durch Hydrieren der entsprechenden ungesättigten Acyloxyverbindungen Ia, Ic und Id erhalten werden.

Als Acyloxyverbindungen kommen Acetate ($R^1$ = $-COCH_3$), Propionate ($R^1$= $-COCH_2CH_3$), Butyrate ($R^1$ = $-COCH_2CH_2CH_3$), Isobutyrate ($R^1$= $-COCH(CH_3)_2$) und Valeriate ($R^1$ $-CHCH_2CH_2CH_2CH_3$) sowie $R^1$ $-COCH_2CH(CH_3)_2$ und $R^1$= $COCH(CH_3)(CH_2CH_3)$ in Betracht.

Die erfindungsgemäßen Verbindungen sind technisch einfach und kostengünstig herstellbar, da die einzusetzenden Aldehyde meist industrielle Zwischenprodukte oder selbst käuflich erhältliche Riechstoffe sind.

Als Beispiele für im 100 t-Maßstab verfügbare Aldehyde seien der Zimtaldehyd sowie der 4-tert.-Butyl-α-methyl-dihydrozimtaldehyd genannt.

Die erfindungsgemäßen Verbindungen der Formel I sind wertvolle Riechstoffe mit frischen blumigen Duftnoten mit langer Haftfestigkeit und großer Stabilität.

**Beispiel 1**

**a) Herstellung einer Vinyl-magnesiumchloridlösung**

In eine Mischung aus 12,2 g (0,5 mol) Magnesiumspänen und 50 ml Tetrahydrofuran (THF) in einem Reaktionskolben wurde langsam Vinylchlorid eingeleitet, wobei sich der Reaktionsansatz auf 60°C erwärmte. Nach Aufnahme von etwa 31 g (0,25 mol) Vinylchlorid wurden noch 100 ml THF zugetropft, anschließend bei Temperaturen von 50 bis 60°C weitere 31 g Vinylchlorid eingeleitet und dann das Reaktionsgemisch bei 50°C noch 30 min nachgerührt.

**b) Herstellung von 1-(4-tert.-Butyl-phenyl)-2-methyl-4-penten-3-ol**

Zu der gemäß 1a) erhaltenen Lösung von Vinylmagnesiumchlorid in THF wurde unter Rühren tropfenweise eine Lösung von 91,8 g (0,45 mol) 3-(4-tert.-Butyl-phenyl)-2-methyl-propanal in gleichem Volumen THF zugefügt, das Reaktionsgemisch anschließend noch 2 Stunden (h) bei 50°C nachgerührt, dann auf 25°C abgekühlt und mit 100 ml Eiswasser hydrolysiert. Anschließend wurde bei 10°C soviel einer 10 % igen wäßrigen Schwefelsäure zugegeben, daß sich der entstandene Niederschlag gerade löste. Die abgetrennte wäßrige Phase wurde noch 2x mit 50 ml Diethylether extrahiert, die vereinigten Extrakte mit Bisulfitlauge, Hydrogencarbonat-Lösung und wenig Wasser gewaschen und nach dem Trocknen der Lösung das Lösungsmittel entfernt.

Der Rückstand wurde bei 0,01 mbar fraktioniert. Man erhielt 92,8 g (0,4 mol) eines farblosen Öls, das bei 151°C/0,01 mbar siedete und einen intensiven blumig-frischen Geruch besaß.

**Beispiel 2**

**1-(4-tert.-Butyl-phenyl)-2-methyl-3-pentanol**

100 g des gemäß Beispiel 1 hergestellten 1-(4-tert.-Butyl-phenyl)-2-methyl-4-penten-3-ols wurde in 100 ml Essigsäureethylester gelöst und in Gegenwart von 10 g eines 0,5 % Palladium auf Aktivkohle-Hydrierkontaktes bei 50°C und 50 bar Wasserstoffdruck hydriert. Nach Destillation (Kp 122°C/0,03 mbar) erhielt man 95 g eines Diastereomerengemisches (45 : 55) an 1-(4-tert.-Butyl-phenyl)-2-methyl-3-pentanol, welches ebenfalls eine interessante blumig-fruchtige Note besitzt.

**Beispiel 3**

**1-(4-tert.-Butyl-phenyl)-2-methyl-4-penten-3-ol-acetat**

Man erhitzt 232 g (1 mol) eines gemäß Beispiel 1 hergestellten 1-(4-tert.-Butyl-phenyl)-2-methyl-4-penten-3-ols mit 204 g (2 mol) Acetanhydrid auf 140°C, wobei man die entstehende Essigsäure gleichzeitig abdestilliert. Das überschüssige Acetanhydrid wurde danach bei leicht vermindertem Druck abdestilliert, der Rückstand abgekühlt, mit 200 ml Diethylether aufgenommen, mit Natriumhydrogencarbonatlösung neutralisiert, der Ether abdestilliert und der Rückstand fraktioniert. Man erhielt 252 g (0,92 mol) eines bei 110°C/0,01 mbar siedenden Öls, das einen intensiven blumigen Geruch aufweist.

**Beispiel 4**

**1-(4-Isopropyl-phenyl)-2-methyl-2,4-pentadien-3-ol**

Analog Beispiel 1b wurde die Lösung von 1 Mol Vinylmagnesiumchlorid in THF mit 169,2 g (0,9 mol) 3-(4-Isopropyl-phenyl)-2-methyl-propenal umgesetzt. Man erhielt 183,6 g (0,85 mol) 1-(4-Isopropyl-phenyl)-2-methyl-1,4-pentadien-3-ol ($Kp_{0,01}$/130°C). Es weist einen intensiven, blumigfrischen Geruch auf, der ausdauernder ist als der von Cyclamenaldehyd.

**Anwendungsbeispiel**

**Seife, enthaltend 1-(4-tert.-Butyl-phenyl)-2-methyl-4 penten-3-ol**

Seifenstücke, die einen erfindungsgemäßen Duftstoff sowie Seifenstücke, die die gleiche Menge des ebenso als Duftstoff verwendbaren 3-(4-tert.-Butyl-phenyl)-2-methyl-propanals enthielten, wurden folgendermaßen hergestellt:

20 g weiße, parfümfreie Seife und 0,1 g des jeweiligen Duftstoffs wurden in einem Porzellangefäß zu einem feinen Pulver verrieben. Hierzu wurden 1,5 l destilliertes Wasser addiert, wodurch eine plastische Masse erzeugt wurde, die zu Stücken gepreßt wurde. Diese wurden Licht und Luft ausgesetzt. Der Geruch der das erfindungsgemäße 1-(4-tert.-Butyl-phenyl)-2-methyl-4-penten-3-ol enthaltenden Seifenstücke blieb während 6 Monaten zart und blumig, während derjenige der das 3-(4-tert.-Butyl-phenyl)-2-methylpropanal enthaltenden Seife nach 6 Monaten eine leicht saure Geruchnote bekam.

**Patentansprüche**

1. 1-Phenyl-2-methyl-alkylverbindungen der allgemeinen Formel I

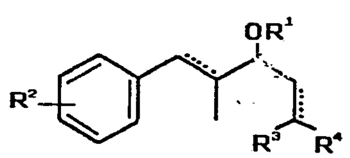

(I),

in der $R^1$ für Wasserstoff oder eine Acylgruppe mit 1 bis 5 C-Atomen vorzugsweise eine Acetylgruppe steht, $R^2$, $R^3$ und $R^4$ gleich oder verschieden sind und für Wasserstoff, eine $C_1$-$C_5$-Alkyl- oder -Alkoxygruppe stehen, wobei die Reste $R^1$, $R^2$, $R^3$ und $R^4$ zusammen maximal 8 C-Atome enthalten sollen und eine oder beide gestrichelten Linien für eine weitere Bindung stehen können.

2. Riechstoff-Kompositionen, enthaltend 1-Phenyl-2-methyl-alkylverbindungen der allgemeinen Formel I gemäß Anspruch 1.

3. Verwendung der 1-Phenyl-2-methyl-alkylverbindungen der allgemeinen Formel I gemäß Anspruch 1 als Riechstoff bzw. als Bestandteile von Riechstoff-Kompositionen.

## Claims

1. A 1-phenyl-2-methylalkyl compound of the formula I

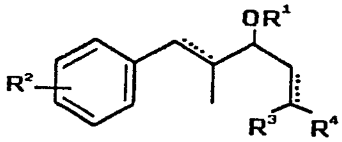

(I)

where $R^1$ is hydrogen or acyl of 1 to 5 carbon atoms, preferably acetyl, $R^2$, $R^3$ and $R^4$ are identical or different and are each hydrogen or $C_1$-$C_5$-alkyl or -alkoxy, and $R^1$, $R^2$, $R^3$ and $R^4$ together should contain not more than 8 carbon atoms and one or both dashed lines may be a further bond.

2. A scent composition containing a 1-phenyl-2-methylalkyl compound of the formula I as claimed in claim 1.

3. Use of a 1-phenyl-2-methylalkyl compound of the formula I as claimed in claim 1 as a scent or as a constituent of a scent composition.

## Revendications

1. Composés 1-phényl-2-méthyl-alkyle de formule générale I

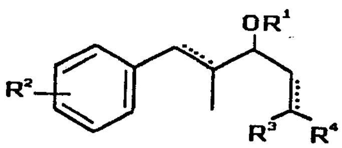

(I)

dans laquelle $R^1$ est mis pour hydrogène ou un groupe acyle ayant 1 à 5 atomes C, de préférence un groupe acétyle, $R^2$ $R^3$ et $R^4$ sont identiques ou différents et sont mis pour hydrogène, un groupe alkyle en $C_1$ -$C_5$ ou un groupe alcoxy, les restes $R^1$, $R^2$, $R^3$ et $R^4$, ensemble, devant contenir au maximum 8 atomes C et une ou les deux lignes pointillées pouvant représenter une autre liaison.

2. Compositions odorantes contenant des composés 1-phényl-2-méthyl-alkyle de formule générale I selon la revendication 1.

3. Utilisation des composés 1-phényl-2-méthyl-alkyle de formule générale 1 selon la revendication 1 comme parfums ou constituants de compositions odorantes.